# EUROPEAN PATENT SPECIFICATION

(11) **EP 0 569 565 B1**
(45) Date of publication and mention of the grant of the patent: **14.08.1996**
(21) Application number: 92922362.6
(22) Date of filing: 01.10.1992
(51) Int. Cl.: A61M 25/02

(54) **A BANDAGE FOR FIXATING A CANNULA OF A VENOUS CATHETER RELATIVE TOA SKIN SURFACE PART OF A PERSON**
BANDAGE ZUM BEFESTIGEN EINER KANÜLE EINES VENENKATHETERS AUF DER HAUTOBERFLÄCHE EINES PATIENTEN
BANDAGE PERMETTANT DE FIXER UNE CANNULE D'UN CATHETER INTRAVEINEUX A LA SURFACE DE LA PEAU D'UN PATIENT

(30) Priority: 29.11.1991 DK 1939/91
(43) Date of publication of application: 18.11.1993
(73) Proprietor: NIKOMED APS, DK-2610 Roedovre (DK)
(72) Inventor: KORNERUP, Niels, DK-2960 Rungsted (DK)
(74) Representative: Nielsen, Henrik Sten
(86) International application number: DK9200289
(87) International publication number: WO9300788

(56) References cited:
- EP-A- 0 168 174
- EP-A- 0 254 696
- EP-A- 0 284 219
- SE-B- 414 994
- SE-B- 419 163

## Description

The present invention relates to a bandage for fixating a cannula of a venous catheter relative to a skin surface part of a person.

A bandage for fixating a cannula of a venous catheter relative to a skin surface part of a person is known from US Patent No. 4.275.721.

The known bandage comprises a support sheet of a foil material and of an oblong configuration. One side surface of the support sheet is provided with an adhesive layer or a sticky layer, on top of which an absorbing pad is arranged. The absorbing pad is located in its entirety within one half of the support sheet, and a rectilinear slit extends from the end or short side of the oblong or rectangularly shaped bandage which end or short side is farthest from the absorbing pad. Basically, this known bandage constitutes a conventional rectangularly shaped plaster having an offset arranged absorbing pad which is circumferentially enclosed or encircled by the adhesive layer, which rectangularly shaped plaster is further provided with a slit which extends from the short side of the rectangularly shaped plaster farthest from the absorbing pad. A conventional plaster may thus easily be converted into this known bandage by simply cutting the conventional plaster into the correct shape so as to locate the absorbing pad in the position offset relative to the centre of the rectangularly shaped bandage and by cutting the slit from the short side of the plaster farthest from the absorbing pad.

As will be readily understood, the bandage known from the above-mentioned US Patent for fixating a cannula of a venous catheter relative to a person is by no means of a structure which has been optimized for its intentional application, i.e. for fixating a cannula of a venous catheter relative to a skin surface part of a patient or person. Thus, this known bandage has to be used in connection with a supporting pad which has to be inserted between the skin surface part of the patient or person and the cannula of the venous catheter, which cannula is to be fixated by means of the bandage. The supporting pad clearly makes it complicated to fixate the cannnula relative to the skin surface part by means of the bandage as not only the cannula has to be kept in a specific position while applying the bandage, but also the supporting pad has to be kept in a desired position supporting the cannula. It has further been realized that this rectangularly shaped, plaster-like bandage known from the above-mentioned US Patent suffers from inherent drawbacks originating from the fact that the overall structure is merely a conventional plaster. Thus, the structure has not been adapted to on the one hand handling the bandage while applying the bandage to the skin surface part of the patient or person for fixating the cannula of a venous catheter and also the supporting pad, if any, and on the other hand, fixating and maintaining the cannula of the venous catheter in a fixed position in order to eliminate any risk of moving the cannula of the venous catheter relative to the supporting skin surface part of the patient or person, which movement might cause damage to the vein of the patient or person, which vein has received the cannula of the venous catheter, or cause other severe damage due to perforation of tissue of the patient or person.

An object of the present invention is to provide a bandage for fixating a cannula of a venous catheter relative to a skin surface part of a person, which bandage is optimized as to on the one hand the handling of the bandage and the cannula while applying the bandage to the cannula and the skin surface part of the person, to which skin surface part the cannula is to be fixated, and on the other hand the fixation of the cannula relative to the person's skin surface part for providing a reliable fixation of the cannula relative to the skin surface part in order to eliminate any risk of causing injuries or harm to the person as a result of unintentional shifting or movement of the cannula relative to the skin surface part.

A particular advantage of the present invention originates from the fact that the bandage according to the present invention may be used without a supporting pad on which the cannula has to rest or has to be supported.

A particular feature of the bandage according to the present invention relates to a highly advantageous absorbing capability due to an enlarged absorbing pad as compared to the above described conventional plaster-like bandage.

The above object, the above advantage, and the above feature together with numerous other objects, advantages, and features which will be evident from the below description are obtained by means of a bandage for fixating a cannula of a venous catheter relative to a skin surface part of a person, which bandage in accordance with the teaching of the present invention comprises:
a support sheet of a foil material having opposite first and second side surfaces and defining an outer rim,
an adhesive layer applied to and covering the first side surface,
an absorbing pad arranged at and adhering to the first side surface, the absorbing pad being located at a distance from the outer rim and being circumferentially encircled by the adhesive layer, characterized in said absorbing pad further being divided into first and second parts, the first part being contiguous to the adhesive layer,
a glue layer applied to the first side surface and covering the first part of the absorbing pad, the second part of the absorbing pad being uncovered by the glue layer, and
a slit cut through the supporting sheet and extending from the outer rim through the adhesive layer and into the first part of the absorbing pad.

In the present context, the term adhesive layer is meant to describe a layer of an adhesive generating a coefficient of adhesion to a stainless steel surface (A.F.E.R.A.: "Association des Fabricants Européens de Rubans Auto-Adhesifs", Test, ref. 4001) less than 1.97 N/cm (5 N/inch), e.g. of the order of 1.54 - 1.97 n/cm (4-5 N/inch), i.e. a fairly weak bond to the skin surface part of the type well-known from conventional plasters.

In the present context, a glue layer is meant to describe a layer of glue generating a coefficient of adhesion to a stainlss steel surface (A.F.E.R.A.: "Association des Fabricants Européens de Rubans Auto-Adhesifs", Test, ref. 4001) of the order of 2.36 - 3.94 N/cm (6-10 N/inch), e.g. of the order of 2.75 - 3.15 N/cm (7-8 N/inch), i.e. an adhesion or a bond stronger than the bond obtained by means of an adhesive layer.

According to the teaching of the present invention, the absorbing pad is divided into two parts, namely a first part covered by the glue layer and generating an absorbing reservoir inserted between the covering glue layer and the support sheet, and an uncovered or exposed part which is to be arranged covering the point of the skin surface part through which point the cannula is inserted into the vein and perforates the tissue. The provision of the glue layer in accordance with the teaching of the present invention firstly establishes a strong bond between the bandage and the supporting skin surface part and consequently an extremely reliable fixation of the bandage relative to the skin surface part. Secondly, the glue layer brings about a strong bond between the support sheet of the bandage and the cannula of the venous catheter and consequently establishes an extremely reliable fixation of the cannula of the venous catheter relative to the skin surface part. Thus, it is to be understood that due to the fact that the slit extends only into the first part of the absorbing pad, the first part of the pad, which part is provided with or covered with the glue layer, establishes the bond between the support sheet and the cannula and further between the support sheet and the skin surface part and establishes a water- and bacteria-tight barrier across the cannula of the venous catheter.

The foil material of the support sheet of the bandage according to the present invention may be made from any appropriate material which may be used as a supporting sheet material. Provided the adhesive layer and the glue layer are substantially water-impermeable, the foil material may be made from water-impermeable or water-permeable material. In order to hinder any penetration of water through the support sheet from the environment into the area covered by the bandage, the foil material is, however, preferably a substantially water-impermeable material.

The bandage may be of any appropriate configuration, such as a polygonal, circular or triangular configuration, however, preferably being of a rectangular configuration. In a highly advantageous embodiment of the bandage according to the present invention, the support sheet, and consequently the overall bandage, is of a quadratic configuration.

Contrary to the above described conventional bandage known from US Patent No. 4.275.721, the absorbing pad of the bandage according to the present invention may be located at any appropriate location at a distance from the outer rim of the support sheet and circumferentially encircled by the adhesive layer. Thus, the absorbing pad of the bandage according to the present invention may be located centrally at the first side surface of the support sheet or alternatively located offset relative to a central position of the first side surface of the support sheet.

Irrespective of the configuration of the support sheet and the overall configuration of the bandage, the absorbing pad of the bandage according to the present invention may be of any appropriate configuration, such as a polygonal, circular, triangular, or preferably a rectangular or quadratic configuration.

In order to amplify the bond between the support sheet and the skin surface part to which the cannula of the venous catheter is to be fixated, the glue layer characteristic of the present invention preferably additionally covers a part of the adhesive layer adjacent to the first part of the absorbing pad.

In order to allow that even the first part of the absorbing pad may absorb liquid, such as blood or water, directly, the glue layer is advantageously perforated.

The glue layer of the bandage according to the present invention may cover a minor area of the first side surface of the support sheet which area may be located at a distance from the outer rim of the support sheet. In the presently preferred embodiment of the bandage according to the present invention, the glue layer constitutes a glue layer strip extending across the first side surface of the support sheet which further increases the bond between the support sheet and the skin surface part and consequently the overall fixation of the bandage and the cannula as well.

In order to render it possible to handle the bandage before the bandage is applied to the cannula and to the skin surface part of the person, to which skin surface part the cannula is to be fixated, the bandage preferably further comprises a release foil. Like a conventional plaster and like the above described bandage known from US Patent No. 4.275.721, the release foil of the bandage according to the present invention is preferably divided into two or more separate sections having individual gripping tabs adjoining one another, e.g. at the absorbing pad or preferably at said first part of said absorbing pad.

Alternatively, the two or more separate sections of the release foil having individual gripping tabs may adjoin one another along a line crossing the slit. Thus, it has been realized that the two or more individual sections of the release foil should preferably adjoin one another at a position shifted away from the centre of the absorbing pad contrary to the structure known from the above-mentioned US patent, as the shifting of the position at which the individual sections of the release foil adjoin one another from the centre of the absorbing pad renders it far more easy to position the absorbing pad correctly relatively to the cannula of the venous catheter, or more precisely relative to the point at which the cannula is introduced into the vein of the person through the skin surface part of the person and to fixate the bandage relative to the skin surface part by removing the release foil.

In the presently preferred embodiment of the bandage according to the present invention, the slit constitutes a straight line, and the line along which the two or more separate sections of the release foil adjoin one another is preferably perpendicular to the slit constituting a straight line or a rectilinear slit.

Provided the support sheet of the bandage is of a rectangular or quadratic configuration, the rectilinear slit preferably extends from a short or long side of the rim of the rectangular or quadratic support sheet, and preferably perpendicular to the rim of the rectangular support sheet.

The present invention will now be further described with reference to the drawings, in which
Fig. 1 is a perspective and schematic view of a first embodiment of a bandage according to the present invention for fixating a cannula of a venous catheter relative to a skin surface part of a person,
Fig. 2 is a perspective view similar to the perspective view of Fig. 1, illustrating the bandage after partly removing cover paper parts of the bandage and disclosing a central adhering, wound-covering and absorbing part of the bandage,
Fig. 3 is a perspective view similar to the perspective view of Fig. 2, illustrating a second embodiment of the bandage according to the present invention,
Fig. 4 is a perspective view illustrating the application of the first embodiment shown in Figs. 1 and 2 of the bandage according to the present invention to the back of a hand of a patient or person to which a cannula of a venous catheter is partially injected and is to be fixated by means of the bandage,
Fig. 5 is a perspective view similar to the perspective view of Fig. 4, illustrating the bandage after a complete application of the bandage to the patient and also illustrating in phantom lines certain parts of the cannula,
Fig. 6 is a sectional and partly cut-away view illustrating in greater details the cannula shown in Figs. 4 and 5, fixated by means of the bandage,
Fig. 7 is a perspective view similar to the views of Figs. 2 and 3, illustrating a third embodiment of the bandage according to the present invention,
Fig. 8 is a perspective view similar to the perspective views of Figs. 4 and 5, illustrating the application of the second embodiment shown in Fig. 3 of the bandage according to the present invention to the back of a patient's or person's hand for fixating a cannula of a venous catheter relative thereto.
Fig. 9 is a perspective and schematic view of a fourth embodiment of the bandage according to the present invention illustrating an alternative slit configuration, and
Fig. 10 is a perspective view similar to the perspective views of Figs. 4, 5, and 8, illustrating the application of the fourth embodiment shown in Fig. 9 of the bandage according to the present invention to the back of a patient's or a person's hand for fixating a cannula of a venous catheter relative thereto.

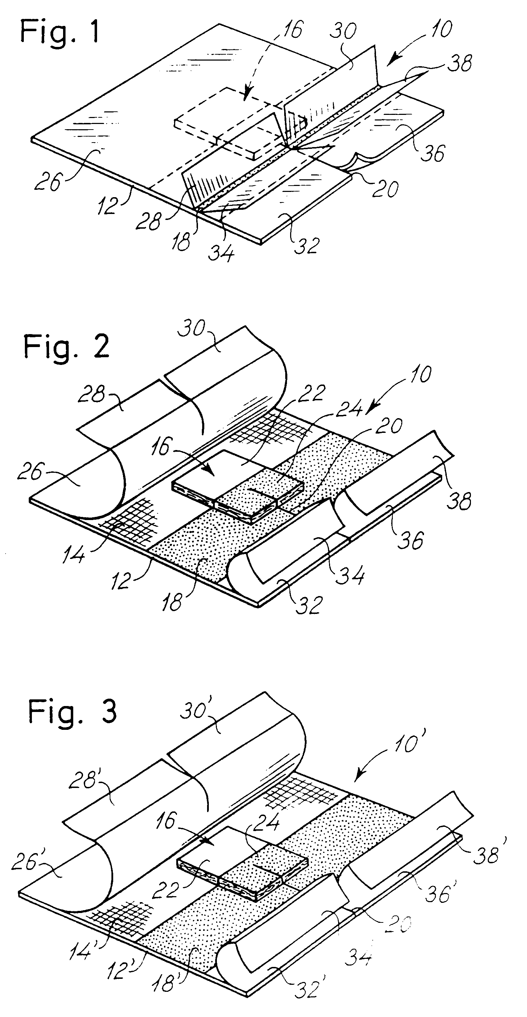

In Figs. 1 and 2, a first embodiment of a bandage for fixating a cannula of a venous catheter relative to a skin surface part of a person is shown designated the reference numeral 10 in its entirety. The skin surface part may e.g. constitute a skin surface part at the back of the person's hand or a skin surface part on the back, forearm, chest or one of the legs of the person in question. The bandage 10 basically comprises a support sheet 12 of a foil material, such as a plastic material, a woven or non-woven fibrous material or any other appropriate foil material and of a substantially quadratic configuration. The sheet 12 is at its one side surface constituting a first side surface to be arranged in contact with and fixated relative to the skin surface part of the person in question, provided with an adhesive layer 14 covering the entire first side surface of the support sheet. On top of the adhesive layer 14, and adhering thereto, an absorbing pad 16 is arranged. The absorbing pad 16 is made from a fibrous material which is an organic-compatible material. The absorbing pad 16 is, as is evident from Figs. 1 and 2, arranged centrally relative to the first side surface of the substantially quadratic support sheet 12. Across the support sheet and across the adhesive layer 14 and the absorbing pad 16, a glue layer 18 extends from one side of the substantially quadratic support sheet 12 to the opposite side of the substantially quadratic support sheet 12. The glue layer 18 may be constituted by a glue layer applied to the support sheet, e.g. by means of an applicator or a spray nozzle, or may comprise an adhesive tape including a carrier foil such as a woven or non-woven fibre material foil impregnated on both sides with a glue or adhesive and differs from the adhesive layer 14 in that the glue of the glue layer 18 provides a far stronger adhesion to the tissue or any other material as compared to the adhesion of the adhesive layer 14. Thus, the glue layer 18 serves the basic purposes of providing a stronger bond to the tissue of the skin surface part of the person, to which skin surface part the bandage 10 is applied so as to provide a stronger and more reliable and lasting fixation of the bandage as compared to a bandage of conventional structure, which conventional bandage is not provided with a glue layer similar to the glue layer 18 and of fixating the cannula of the venous catheter relative to the skin surface part of the person, as will be evident from the description below. The absorbing pad 16 is divided into two parts designated the reference numerals 22 and 24, respectively, which parts constitute a part uncovered by the glue layer 18 and a part covered by the glue layer 18, respectively. Thus, it is to be emphasized that the glue layer 18 serves the additional or supplementary purpose of providing a strong and reliable fixation of the absorbing pad relative to the skin surface part of the person to which the bandage 10 is applied in order to eliminate any risk that the absorbing pad 16 during the application of the bandage or after the bandage has been applied to the skin surface, is shifted from a specific position relative to a perforated point of the skin surface part at which the uncovered part 22 of the absorbing pad 16 is arranged.

In order to render it possible to arrange the bandage 10 enclosing the cannula of the venous catheter and further to arrange the uncovered part 22 of the absorbing pad 16 correctly and adequately relative to the perforated point of the skin surface part of the person in question, a slit 20 is provided, which slit extends from a side of the substantially quadratic support sheet 12 perpendicular to the glue layer 18 and through the glue layer 18 and into the absorbing pad 16. The slit 20, however, extends into the part 24 of the absorbing pad 16 exclusively and ends at a small distance, such as a distance of the order of 3-5 mm from the boundary line between the parts 22 and 24 of the absorbing pad 16.

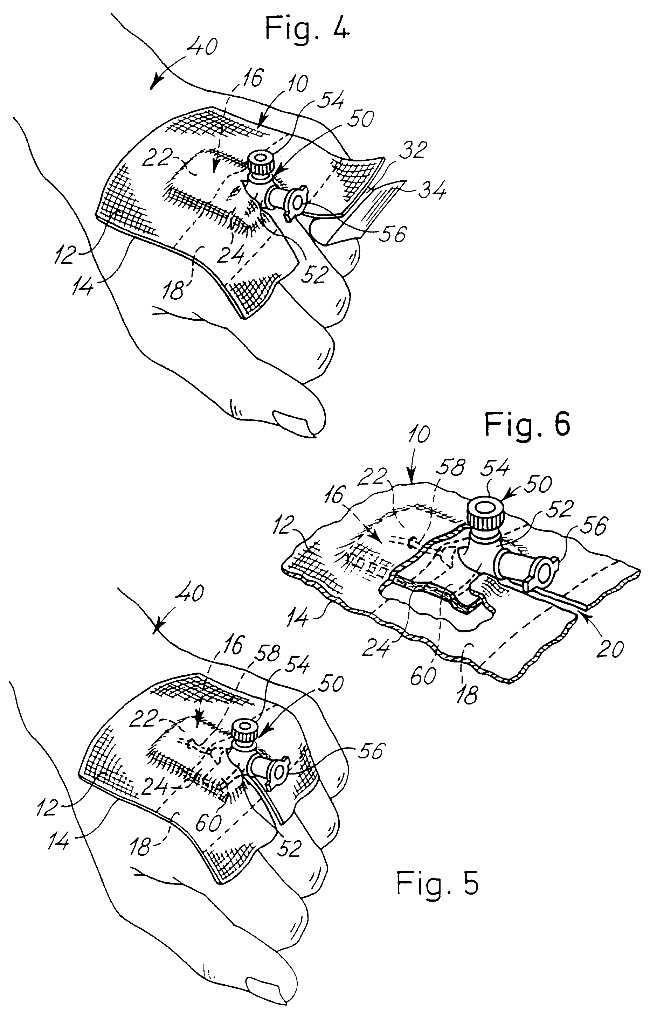

The adhesive layer 14, the uncovered part 22 of the absorbing pad 16, and the glue layer 18 are covered by a total of three sheets of a release foil designated the reference numeral 26, 32, and 36. The sheet 26 constitutes a major sheet which covers the major surface part of the first side surface of the support sheet and extends from a side of the substantially quadratic support sheet 12, which side is opposite to the side from which the slit 20 extends into the glue layer 18, and covers not only the major part of the adhesive layer 14, the uncovered part 22 of the absorbing pad 16, but also approximately one half of the glue layer 18. Thus, as is evident from Figs. 1 and 2, the slit 20 also divides or partly separates the sheet 26 and divides a gripping tab of the sheet 26 into two separate gripping tabs 28 and 30. The two remaining release foil sheets 32 and 36 have gripping tabs, designated the reference numerals 34 and 38, respectively. The sheets 26 and 32,36 meet one another along a line extending along the glue layer 18 approximately at the central line of the glue layer 18, as is evident from Fig. 1. The provision of a total of three release foil sheets meeting one another along a line extending along the glue layer 18 provides a highly advantageous feature as to the application of the bandage 10, as will be discussed below with reference to Figs. 4 and 5.

In Fig. 1, the release foil sheets 26, 32, and 36 are shown arranged in contact with and covering the adhesive layer 14, the glue layer 18, and the absorbing pad 16, whereas in Fig. 2, the release foil sheets 26, 32, and 36 are partly removed from the bandage 10 disclosing the adhesive layer 14, the glue layer 18, and further the absorbing pad 16, i.e. the adhering, wound-covering and absorbing parts of the bandage to be applied or arranged in contact with the skin surface part of the person in question to which skin surface part a cannula of a venous catheter is to be fixated.

In Figs. 4 and 5, a hand 40 of a person or a patient is shown. More precisely, the back of the hand is shown, to which a cannula assembly 50 is applied or arranged. The cannula assembly 50 is basically of a conventional structure and comprises an assembly body 52 from which a needle extends, which needle is shown in phantom lines in Fig. 5 and designated the reference numeral 58. The assembly body 52 further receives two plugs 54 and 56, which plug 54 serves the purpose of blocking an opening of the assembly body 52 and of allowing the administration or introduction of a medicament or a drug to the person or patient. The plug 54 serves the purpose of blocking an opening of the assembly body 52, to which opening the venous catheter is to be connected. The cannula assembly 50 further comprises two wing components, one of which is shown in phantom lines in Fig. 6 and designated the reference numeral 60. The wings, such as the wing 60, serve the purpose of preventing the cannula assembly 50 from tilting as the cannula assembly is arranged in contact with the skin surface of the back of the person's hand after the needle 58 has been introduced into the vein at the back of the person's hand.

After the needle 58 has been introduced into the vein, the bandage 10 is applied on top of the cannula assembly 50, as is illustrated in Fig. 4, in order to firstly cover the point of the skin surface part of the person, through which point the needle 58 is introduced into the vein and perforates the tissue, secondly of fixating the cannula assembly 50 relative to the back of the person's hand, and thirdly of sealing said point through which the needle is introduced into the vein in order to exclude bacteria from the environment from getting into the vein and causing infections.

The bandage 10 is preferably applied in the following way. The part 22 of the absorbing pad 16 is arranged above the point of the person's skin surface, through which point the needle 58 perforates the tissue, and the entire bandage 10 is arranged in a position, so to speak straddling the cannula assembly 50. The release foil sheet 26 is now removed by gripping one of the gripping tabs 28 and 30 and by pulling off the release foil sheet 26, while still maintaining the uncovered or exposed part 22 of the absorbing pad 16 in the intentional correct position.

The needle 58 and the body 52 of the cannula assembly 50 is now partly fixated by pressing the uncovered part of the glue layer 18 into contact with the needle 58 and the body 52 of the cannula assembly 50 and further into intimate contact with the outer side surface of the person for fixating the bandage 10 relative to the person's skin. Thereupon, the release foil sheets 32 and 36 are removed one at a time, which renders it possible to fixate the wings of the body 52 of the cannula assembly 50 properly and correctly by simply tearing off the release foil sheet and pressing down the bandage against the back of the person's hand. In Fig. 4, the stage of eventually removing the release foil sheet 32 is disclosed, whereas in Fig. 5, the entire bandage 10 is correctly applied and fixated for fixating the cannula assembly 50.

In Fig. 6, an enlarged, partly broken away view is shown illustrating the cannula assembly 50 and the adjacent parts of the bandage 10. It is to be emphasized that contrary to conventional bandages of this type, the bandage according to the present invention, as is evident from Fig. 6, need not be used in connection with a separate pad which has to be positioned below the wings, such as the wing 60 of the body 52 of the cannula assembly 50, as the strong bond between the glue layer 18 of the bandage 10 according to the present invention and the needle 58, the body 52 and further the tissue of the person eliminates the need of the employment of a separate absorbing pad to be arranged below the cannula assembly 50 as a liquid- and bacteria-tight barrier is provided by the glue layer 18 across the needle 58 and the body 52. In some cases, the person's or patient's tissue, however, need to be protected from coming into contact with the fairly rigid cannula assembly body 52, and a separate supporting pad may under these circumstances be employed.

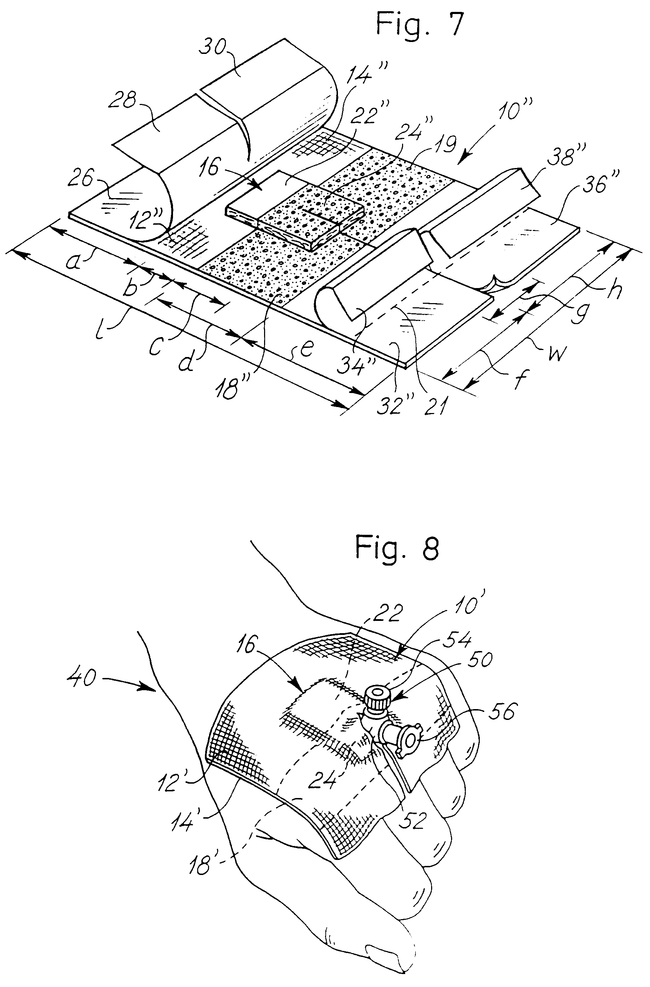

In Figs. 3 and 7, second and third embodiments, respectively, of the bandage according to the present invention are shown, which embodiments are basically of a structure similar to the structure of the first embodiment of the bandage according to the present invention described above with reference to Figs. 1 and 2. Thus, the second embodiment shown in Fig. 3 basically differs from the above described first embodiment in that the bandage is of a somewhat different geometrical configuration as the support sheet is of a rectangular configuration. Apart from this geometric difference, the second embodiment shown in Fig. 3 is identical to the first embodiment discussed above with reference to Figs. 1 and 2. The second embodiment of the bandage according to the present invention shown in Fig. 3 is in its entirety designated the reference numeral 10', and components or parts of the second embodiment 10' of the bandage according to the present invention, which components or parts differ from similar components of the first embodiment 10 of the bandage according to the present invention described above with reference to Figs. 1 and 2 in the geometrical shape or dimensions exclusively are designated the same reference numerals as the corresponding component or part of the first embodiment 10, however, supplemented with a '. Thus, the support sheet of the second embodiment 10' shown in Fig. 3, which support sheet corresponds to the support sheet 12 shown in Figs. 1 and 2, is designated the reference numeral 12'. Similarly, the components or parts 14', 18', 26', 28', 30', 32', 34', 36', and 38' of the second embodiment 10' of the bandage according to the present invention correspond to the components 14, 18, 26, 28, 30, 32, 34, 36, and 38, respectively, shown in Figs. 1 and 2 and discussed above with reference thereto.

In Fig. 8, the second embodiment 10' of the bandage according to the present invention is shown applied to the hand 40 of the person or patient to which the cannula assembly 50 is applied. By comparing Figs. 4, 5, and Fig. 8, the increased width of the second embodiment 10' as compared to the substantially quadratic first embodiment 10 shown in Figs. 4 and 5 is evident.

In Fig. 7, a third embodiment of the bandage according to the present invention is shown, which bandage is designated the reference numeral 10''. Like the second embodiment described above with reference to Fig. 3, components or parts of the third embodiment 10'' of the bandage according to the present invention, which components or parts correspond to components or parts of the first embodiment 10 of the bandage according to the present invention described above with reference to Figs. 1 and 2 are designated the same numerals, however, added a ''. The third embodiment of the bandage 10'' shown in Fig. 7 differs from the first embodiment 10 shown in Figs. 1 and 2 in that the support sheet 12 is of a rectangular configuration. Furthermore, whereas in the second embodiment 10' of the bandage according to the present invention shown in Fig. 3, the absorbing pad 22 is arranged centrally relative to the support sheet 12' and the slit 20 extends from one of the long sides of the rectangularly shaped support sheets 12', the absorbing pad 16 of the third embodiment 10'' of the bandage according to the present invention shown in Fig. 7 is arranged offset relative to the centre of the support sheet 12''. Furthermore, the second embodiment 10'' constitutes an elongated structure as the slit 20 extends from one of the short sides of the rectangularly shaped support sheet 12'' which short side constitutes the short side relative to which the absorbing pad 16 is arranged at the longest distance. The third embodiment 10'' of the bandage according to the present invention shown in Fig. 7 further differs from the above described first and second embodiments of the bandage according to the present invention in that the glue layer 18'' is a perforated glue layer, as a number of holes 19 extending through the glue layer are provided. The perforation of the glue layer 18'' serves the purpose of rendering the second part 24'' of the absorbing pad 16 at least partially absorbing, so that the second part 24'' of the absorbing pad 16 may absorb liquid, such as blood, as the bandage 10'' is arranged on the back of a person's hand, as disclosed in Figs. 4, 5, and 8.

In Fig. 7, the dimensions of the support sheet 12'' are indicated as the length l and the width w. The spacing or distances between the different components or parts of the bandage 10'' are designated references a, b, c, d, e, f, g, and h. These dimensions or distances are also applicable to the first embodiment 10 shown in Figs. 1 and 2, and the second embodiment 10' shown in Fig. 3.

In Fig. 7, a dotted line 21 is further shown, which dotted line may be marked on the release foil sheets 32'' and 36'' and indicating a cutting line along which the bandage 10'' may be cut, e.g. by means of scissors, so as to convert the elongated, rectangularly shaped bandage 10'' into a quadratic bandage in which the absorbing pad 16 is arranged centrally relative to the support sheet 12''. Consequently, by reducing the length l of the entire bandage 10'' to the dimension w, the third embodiment 10'' shown in Fig 7 is modified into a bandage similar to the first embodiment 10 shown in Figs. 1 and 2, however, differing from the first embodiment 10 in that the glue layer 18'' is perforated.

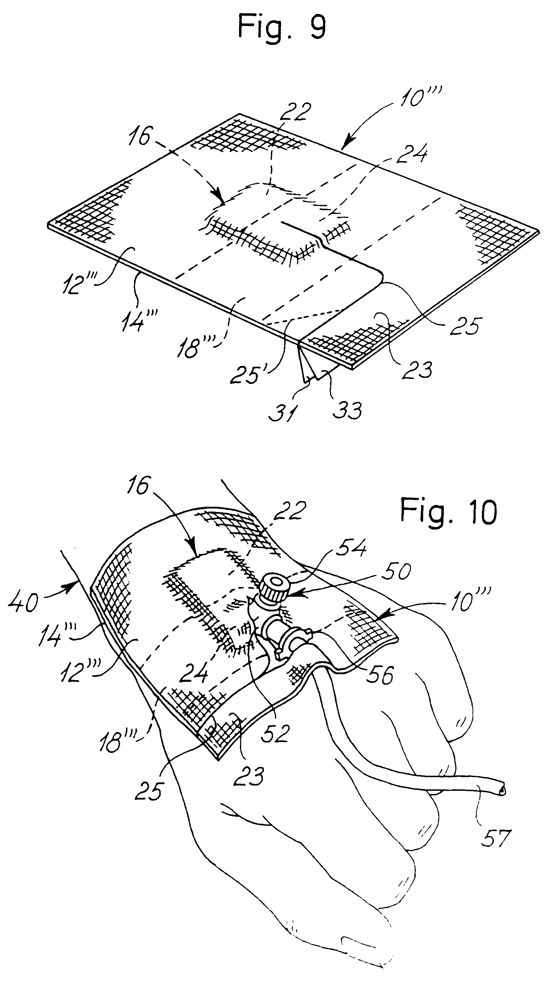

In Fig. 9, a fourth embodiment of the bandage according to the present invention is shown, which embodiment is basically of a structure similar to the structure of the first embodiment of the bandage according to the present invention described above with reference to Figs. 1 and 2. Fig. 9 discloses a side surface of the fourth embodiment of the bandage opposite to the side surface of the first, second, and third embodiments shown in Figs. 1 and 2, 3, and 7, i.e. a second side surface opposite to the first side surface to be arranged in contact with the skin surface part of the patient or person and consequently facing outwards. The fourth embodiment shown in Fig. 9 basically differs from the above described first embodiment in that the slit is of a different configuration as compared to the rectilinear slit 20 shown in Figs. 1 and 2. The fourth embodiment of the bandage according to the present invention shown in Fig. 9 is in its entirety designated the reference numeral 10''', and components or parts of the fourth embodiment 10''' of the bandage according to the present invention, which components or parts differ from similar components of the first embodiment 10 of the bandage according to the present invention described above with reference to Figs. 1 and 2 in the geometrical shape or dimensions exclusively, are designated the same reference numerals as the corresponding component or part of the first embodiment 10, however, supplemented with a '''. Thus, the components or parts 12''', 14''', and 18''' of the fourth embodiment 10''' of the bandage according to the present invention correspond to the components 12, 14, and 18, respectively, shown in Figs. 1 and 2 and discussed above with reference thereto. A non-rectilinear slit designated the reference numeral 25 is shown in Fig. 9, which slit serves the purpose of providing a tab 23 of the support foil 12, which tab may serve the purpose of fixating a tubing of the venous catheter as will be described below with reference to Fig. 10. Similarly, the release foils 32 and 36 are combined into a single release foil having a single gripping tab 33. The release foil 26 is similarly enlarged and provided with a gripping tab 31, which gripping tab 31 meets the gripping tab 33 along a rectilinear segment of the slit 25, which rectilinear segment extends parallel with the glue layer 18'''. The reference numeral 25' designates an alternative configuration of the non-rectilinear slit 25.

In Fig. 10, the fourth embodiment 10''' of the bandage according to the present invention is shown applied to the back of the hand 40 of the patient or person to which the cannula assembly 50 is applied. In Fig. 10, the cannula assembly 50 is further provided with a tubing 57 which is fixed to the plug 56 and further fixated relative to the back of the hand 40 by means of the tab 23 of the support foil 12''' of the fourth embodiment 10''', which tab 23 is applied across the tubing 57.

It is to be realized that the above described embodiments may be modified in numerous ways and also be combined by providing a specific component of one of the embodiments in a different embodiment of the bandage.

### Example 1

The bandage shown in Fig. 7, however, converted into a quadratic configuration, was made from the following materials: the support sheet 12'' was made from a non-woven 100% polyester foil of the type CHICOPEE (TM), Keyback Style of a density of approx. 62 g/m² and of a thickness of approx. 300 µm. The length l and width w of the support sheet 12'' was 72 mm and 72 mm, respectively. The adhesive layer 14'' was a medical acrylic adhesive, and the glue layer 18'' was a synthetic rubber resin adhesive applied to both sides of a carrier strip of low density, non-woven polyester foil. The perforation holes 19 were constituted by cuts or slits of a length of approx. 2 mm. The absorbing pad 16 measured (b + c) x g: 28 mm x 21 mm and was made from 100% cotton fibres. The release foil from which the sheets 26, 32, and 36 were made was a siliconized paper foil of a density of approx. 100 g/m² and a thickness of approx. 100 µm. The width d of the glue layer 18 was 20 mm, the dimension b was 12 mm, and the dimension c was 16 mm. The distance a was 22 mm, and the dimension e was 18 mm. The dimensions f and h were both 36 mm.

### Example 2

Alternative embodiments corresponding to the embodiments 10 and 10' shown in Figs. 1, 2, and 3, respectively, may be made from the same materials as discussed above. The length and the width of the support sheet 12 or 12' may be of the order 80 mm x 70 mm, 75 mm x 70 mm, 80 mm x 75 mm, 95 mm x 70 mm. The dimension a is preferably of the order of 20-25 mm, the ratio b/c is preferably equal to or less than 1, the distance e is preferably of the order of 15-30 mm, and the ratio (b + c)/g is preferably larger than 1, however, less than 2. The dimensions f and h are preferably identical.

### Example 3

Alternative materials which may be interesting or relevant for the production of the bandage according to the present invention are: The support sheet may be made from spun bond, spun lace, woven or non-woven foil materials of polyester, nylon, polypropylene, polyethylene, polyurethane, polyvinylchloride, clear tape or other transparent or non-transparent material, or combinations thereof, e.g. providing a separate transparent area adjacent to the absorbing pad through which transparent area the tip of the needle of the venous catheter may be viewed or disclosed. Preferably, the support sheet is made from a foil material of a thickness of 150-400 µm. The adhesive layer may be constituted by a medical, natural or synthetic rubber resin adhesive or other medical grade adhesive. The release foil may be constituted by a siliconized or non-siliconized polyester, polypropylene, polyethylene or similar plastic material. Preferably, the release foil is of a thickness of 70-150 µm. The absorbing pad may be made from cross-laid, fibre-bonded viscose-rayon or polyester material, including polyolefine and similar absorbent fibre material. The glue layer may be made from e.g. a polyester tape provided with a medical acrylic adhesive or medical synthetic rubber resin on both sides. Alternatively, any strong bond medical grade adhesive may be used, providing a glue layer supported by a carrier of woven or non-woven fibre material, such as polyester fibre material or the like.

Although the present invention has been described above with reference to specific, presently preferred embodiments of the bandage according to the present invention, the present invention is, however, by no means to be construed limited to the above embodiments, as numerous modifications or amendments are readily perceivable by a person having ordinary skill in the art, within the scope of the present invention as defined in the appended claims.

## Claims

1. A bandage (10, 10', 10'', 10''') for fixating a cannula of a venous catheter relative to a skin surface part of a person, comprising:
a support sheet (12, 12', 12'', 12''') of a foil material having opposite first and second side surfaces and defining an outer rim,
an adhesive layer (14) applied to and covering said first side surface,
an absorbing pad (16) arranged at and adhering to said first side surface, said absorbing pad (16) being located at a distance from said outer rim and being circumferentially encircled by said adhesive layer (14),
a slit (20) cut through said supporting sheet (12, 12', 12'', 12''') and extending from said outer rim through said adhesive layer (14),
**CHARACTERIZED** in
said absorbing pad (14) further being divided into first and second parts (22, 24, 22'', 24''), said first part (24, 24'') being contiguous to said adhesive layer (14),
a glue layer (18, 18', 18'') applied to said first side surface and covering said first part (24, 24'') of said absorbing pad (16), said second part (22, 22'') of said absorbing pad (16) being uncovered by said glue layer (18, 18', 18''), and
said slit extending into said first part (24, 24'') of said absorbing pad (16).

2. A bandage according to Claim 1, said foil material being a water-impermeable material.

3. A bandage according to any of the Claims 1-2, said support sheet (12, 12', 12'', 12''') being of a rectangular configuration.

4. A bandage according to any of the Claims 1-3, said support sheet (12, 12', 12'', 12''') being of a quadratic configuration.

5. A bandage according to any of the Claims 1-4, said absorbing pad (16) being located centrally at said first side surface of said support sheet (12, 12', 12'', 12''').

6. A bandage according to any of the Claims 1-4, said absorbing pad (16) being located offset relative to a central position at said first side surface of said support sheet (12, 12', 12'', 12''').

7. A bandage according to any of the Claims 1-6, said absorbing pad (16) being of a rectangular configuration.

8. A bandage according to any of the Claims 1-6, said absorbing pad (16) being of a quadratic configuration.

9. A bandage according to any of the Claims 1-8, said glue layer additionally covering a part of said adhesive layer adjacent to said first part (24, 24'') of said absorbing pad (16).

10. A bandage according to any of the Claims 1-9, said glue layer (18, 18', 18''') being perforated.

11. A bandage according to any of the Claims 9-10, said glue layer (18, 18', 18'', 18''') constituting a glue layer strip extending across said first side surface of said support sheet (12, 12', 12'', 12''').

12. A bandage according to any of the Claims 1-11, further comprising a release foil.

13. A bandage according to Claim 12, said release foil being divided into two or more separate sections having individual gripping tabs (28, 28', 30, 30', 34, 34', 38, 38') adjoining one another at said absorbing pad (16).

14. A bandage according to Claim 13, said two or more separate sections of said release foil preferably adjoining one another at said first part (24, 24'') of said absorbing pad (16).

15. A bandage according to any of the Claims 12-13, said release foil being divided into two or more separate sections having individual gripping tabs adjoining one another along a line crossing said slit (20).

16. A bandage according to any of the Claims 1-15, said slit (20) being rectilinear.

17. A bandage according to the Claims 15 and 16, said line being perpendicular to said rectilinear slit (20).

18. A bandage according to Claims 3 and 17, said rectilinear slit (20) extending from a short side of said rim of said rectangular support sheet (12, 12', 12'', 12''').

19. A bandage according to Claims 3 and 17, said rectilinear slit (20) extending from a long side of said rim of said rectangular support sheet (12, 12', 12'', 12''').

## Patentansprüche

1. Bandage (10,10',10'',10''') zum Befestigen einer Kanüle eines Venenkatheters auf der Hautoberfläche eines Patienten, mit:
- einer Trägerfolie (12,12',12'',12''') aus einem Folienmaterial, die eine erste und eine dieser gegenüberliegende zweite Seilenfläche hat und einen äußeren Rand definiert;
- einer Haftschicht (14), die auf die erste Seitenfläche diese bedeckend aufgebracht ist;
- einem Saugkissen (16), das an der ersten Seitenfläche an dieser haftend angeordnet ist, mit Abstand zu dem äußeren Rand liegt und in Umfangsrichtung von der Haftschicht (14) umgeben ist; und
- einem Schlitz (20), der durch die Trägerfolie (12,12',12'',12''') geschnitten ist und von dem äußeren Rand durch die Haftschicht (14) verläuft,
dadurch **gekennzeichnet,** daß
- das Saugkissen (16) außerdem in einen ersten und einen zweiten Abschnitt (22,24,22'',24'') unterteilt ist, wobei der erste Abschnitt (24,24'') zu der Haftschicht (14) benachbart ist;
- eine Klebstoffschicht (18,18',18'') auf die erste Seiterfläche aufgebracht ist und den ersten Abschnitt (24,24'') des Saugkissens (16) bedeckt, wobei der zweite Abschnitt (22, 22'') des Saugkissens (16) von der Klebstoffschicht (18,18',18'') unbedeckt bleibt; und
- der Schlitz (20) bis in den ersten Abschnitt (24,24'') des Saugkissens (16) verläuft.

2. Bandage nach Anspruch 1, dadurch **gekennzeichnet,** daß das Folienmaterial ein wasserdichtes Material ist.

3. Bandage nach Anspruch 1 oder 2, dadurch **gekennzeichnet,** daß die Trägerfolie (12,12',12'',12''') eine rechteckige Form hat.

4. Bandage nach einem der Ansprüche 1 bis 3, dadurch **gekennzeichnet,** daß die Trägerfolie (12,12',12'',12''') eine quadratische Form hat.

5. Bandage nach einem der Ansprüche 1 bis 4, dadurch **gekennzeichnet,** daß das Saugkissen (16) in der Mitte der ersten Seitenfläche der Trägerfolie (12,12',12'', 12''') liegt.

6. Bandage nach einem der Ansprüche 1 bis 4, dadurch **gekennzeichnet,** daß das Saugkissen (16) versetzt in Bezug auf die Mitte der ersten Seitenfläche der Trägerfolie (12,12',12'', 12''') liegt.

7. Bandage nach einem der Ansprüche 1 bis 6, dadurch **gekennzeichnet,** daß das Saugkissen (16) eine rechteckige Form hat.

8. Bandage nach einem der Ansprüche 1 bis 6, dadurch **gekennzeichnet,** daß das Saugkissen (16) eine quadratische Form hat.

9. Bandage nach einem der Ansprüche 1 bis 8, dadurch **gekennzeichnet,** daß die Klebstoffschicht zusätzlich einen an den ersten Abschnitt (24,24'') des Saugkissens (16) grenzenden Abschnitt der Haftschicht bedeckt.

10. Bandage nach einem der Ansprüche 1 bis 9, dadurch **gekennzeichnet,** daß die Klebstoffschicht (18,18',18''') perforiert ist.

11. Bandage nach Anspruch 9 oder 10, dadurch **gekennzeichnet,** daß die Klebstoffschicht (18,18',18'',18''') einen Klebstoffschichtstreifen bildet, der über die erste Seitenfläche der Trägerfolie (12,12',12'',12''') verläuft.

12. Bandage nach einem der Ansprüche 1 bis 11, **gekennzeichnet** durch eine Trennfolie.

13. Bandage nach Anspruch 12, dadurch **gekennzeichnet,** daß die Trennfolie in wenigstens zwei separate Bereiche mit einzelnen Greiffahnen (28,28',30,30',34,34', 38,38') unterteilt ist, die an dem Saugkissen (16) aneinanderstoßen.

14. Bandage nach Anspruch 13, dadurch **gekennzeichnet,** daß die wenigstens zwei separaten Bereiche der Trennfolie bevorzugt an dem ersten Abschnitt (24,24'') des Saugkissens (16) aneinanderstoßen.

15. Bandage nach Anspruch 12 oder 13, dadurch **gekennzeichnet,** daß die Trennfolie in wenigstens zwei separate Bereiche mit einzelnen Greiffahnen unterteilt ist, die entlang einer den Schlitz (20) kreuzenden Linie aneinanderstoßen.

16. Bandage nach einem der Ansprüche 1 bis 15, dadurch **gekennzeichnet,** daß der Schlitz (20) geradlinig ist.

17. Bandage nach den Ansprüchen 15 und 16, dadurch **gekennzeichnet,** daß die Linie rechtwinklig zu dem geradlinigen Schlitz (20) ist.

18. Bandage nach den Ansprüchen 3 und 17, dadurch **gekennzeichnet,** daß der geradlinige Schlitz (20) von einer kurzen Seite des Randes der rechteckigen Trägerfolie (12,12',12'',12''') wegläuft.

19. Bandage nach den Ansprüchen 3 und 17, dadurch **gekennzeichnet,** daß der geradlinige Schlitz (20) von einer langen Seite des Randes der rechteckigen Trägerfolie (12,12',12'',12''') wegläuft.

## Revendications

1. Bandage (10,10',10'',10''') pour fixation d'une canule d'un cathéter veineux à une partie de surface de la peau d'une personne, comprenant :
un élément porteur (12,12',12'',12''') en une matière en feuille qui présente une première et une deuxième surfaces latérales opposées et qui définit un bord extérieur,
une couche adhésive (14) appliquée à ladite première surface latérale et couvrant celle-ci,
un tampon absorbant (16) placé sur ladite première surface latérale et adhérant à celle-ci, ledit tampon absorbant (16) étant situé à distance dudit bord extérieur et étant circonférentiellement entouré par ladite couche adhésive (14), et
une fente (20) coupée à travers ledit élément porteur en feuille (12,12',12'',12''') et s'étendant à partir dudit bord extérieur, à travers ladite couche adhésive (14), caractérisé en ce que :
ledit tampon absorbant (14) est en outre divisé en une première et une deuxième parties (22,24,22'',24''), ladite première partie (24,24'') étant contiguë à ladite couche adhésive (14),
une couche de colle (18,18',18'') est appliquée à la dite première surface latérale et couvre ladite première partie (24,24'') dudit tampon absorbant (16), ladite deuxième partie (22,22'') dudit tampon absorbant (16) n'étant pas couverte par ladite couche de colle (18,18',18''), et
ladite fente s'étend dans ladite première partie (24,24'') dudit tampon absorbant (16).

2. Bandage suivant la revendication 1, ladite matière en feuille étant une matière imperméable à l'eau.

3. Bandage suivant une quelconque des revendications 1 et 2, ledit élément support en feuille (12,12', 12'',12''') étant de configuration rectangulaire.

4. Bandage suivant une quelconque des revendications 1 à 3, ledit élément support en feuille (12,12',12'', 12''') étant de configuration quadratique.

5. Bandage suivant une quelconque des revendications 1 à 4, ledit tampon absorbant (16) étant placé centralement sur ladite première surface latérale dudit élément porteur en feuille (12,12',12'',12''').

6. Bandage suivant une quelconque des revendications 1 à 4, ledit tampon absorbant (16) étant décalé par rapport à une position centrale sur ladite première surface latérale dudit élément porteur en feuille (12,12',12'', 12''').

7. Bandage suivant une quelconque des revendications 1 à 6, ledit tampon absorbant (16) étant de configuration rectangulaire.

8. Bandage suivant une quelconque des revendications 1 à 6, ledit tampon absorbant (16) étant de configuration quadratique.

9. Bandage suivant une quelconque des revendications 1 à 8, ladite couche de colle couvrant en outre une partie de ladite couche adhésive adjacente à ladite première partie (24,24'') dudit tampon absorbant (16).

10. Bandage suivant une quelconque des revendications 1 à 9, ladite couche de colle (18,18',18''') étant perforée.

11. Bandage suivant une quelconque des revendications 9 et 10, ladite couche de colle (18,18',18'',18''') constituant une bande de couche de colle s'étendant en travers de ladite première surface latérale dudit élément porteur en feuille (12,12',12'',12''').

12. Bandage suivant une quelconque des revendications 1 à 11, comprenant en outre une pellicule anti-adhérence.

13. Bandage suivant la revendication 12, ladite pellicule anti-adhérence étant divisée en deux parties séparées, ou plus, comportant des languettes de préhension individuelles (28,28',30,30',34,34',38,38') mutuellement adjacentes à l'endroit dudit tampon absorbant (16).

14. Bandage suivant la revendication 13, les dites deux parties séparées ou plus de ladite pellicule anti-adhérence étant de préférence mutuellement adjacentes à l'endroit de ladite première partie (24,24'') dudit tampon absorbant (16).

15. Bandage suivant une quelconque des revendications 12 et 13, ladite pellicule anti-adhérence étant divisée en deux parties séparées ou plus comportant des languettes de préhension individuelles mutuellement adjacentes le long d'une ligne qui croise ladite fente (20).

16. Bandage suivant une quelconque des revendications 1 à 15, ladite fente (20) étant rectiligne.

17. Bandage suivant les revendications 15 et 16, ladite ligne étant perpendiculaire à ladite fente rectiligne (20).

18. Bandage suivant les revendications 3 et 17, ladite fente rectiligne (20) s'étendant à partir d'un petit côté dudit bord dudit élément porteur rectangulaire en feuille (12,12',12'',12''').

19. Bandage suivant les revendications 3 et 17, ladite fente rectiligne (20) s'étendant à partir d'un grand côté dudit bord dudit élément porteur rectangulaire en feuille (12,12',12'',12''').
